# EUROPEAN PATENT SPECIFICATION

(11) **EP 1 173 701 B1**
(45) Date of publication and mention of the grant of the patent: **06.09.2006**
(21) Application number: 00929137.8
(22) Date of filing: 26.04.2000
(51) Int. Cl.: F16L 55/162, A61M 29/00

(54) **INTRODUCER AND PLACER OF REPAIRS IN TUBULATIONS**
VORRICHTUNG ZUM EINFÜHREN UND ANBRINGEN VON REPARATURELEMENTEN IN ROHREN
DISPOSITIF D'INSERTION ET D'IMPLANTATION D'ÉLÉMENTS POUR LES RÉPARATIONS DANS DES TUBES

(30) Priority: 26.04.1999 BR 9900959
(43) Date of publication of application: 23.01.2002
(73) Proprietor: Teixeira Moreira, Luciano José, CEP-88036-560 Florianopolis SC (BR); Machado Peres, Ricardo, CEP-88040-900 Florianopolis SC (BR); Silveira, Pierre Galvani, CEP-88020-620 Centro Florianopolis SC (BR)
(72) Inventor: Teixeira Moreira, Luciano José, CEP-88036-560 Florianopolis SC (BR); Machado Peres, Ricardo, CEP-88040-900 Florianopolis SC (BR); Silveira, Pierre Galvani, CEP-88020-620 Centro Florianopolis SC (BR)
(74) Representative: Elzaburu Marquez, Alberto
(86) International application number: PCT/BR2000/000042
(87) International publication number: WO 2000/065270

(56) References cited:
- US-A- 5 683 451

## Description

Mechanical device, tubular, with compartment to store tubulation repair and with commands to introduce and to place the repair in a pre-determined point in the tubulation internal wall

The repair services in hard-accessed tubulations, has been a constant worry to the involved professionals. The leakage caused by perforation in a mansory built-in hydraulic tubulation has required the rupture and withdrawal of part of the wall's revetment. It could be repaired accessing the tubulation's internal wall, through one of the ends. The device proposed in this report is also an alternative in cases of chimney of small diameters, with linkages in specific points throughout the tubulation and it is hard to access through the tubulation's external wall to make a repair. In the medicine field, there is the need of putting repairs, called prothesis, inside the tubulation of the digestive and vascular systems; in this field, there are several types of devices, called catheter, destined to introduction and placement of the prothesis in specific points of the internal wall of veins and arteries.

Patent US 5,683,451 is related to same group of catheter that pushes the prosthesis out of it. It includes a plurality of disposed runners 42 affixed together at one of their proximal ends to one of the shaft 34 ends, which push the prosthesis out of the catheter. The runners 34 remain around the prosthesis 10 reducing its sliding resistance related to the sheath 32 inner wall. Doing this the desired technical effect is withdrawing prosthesis 10 out of inside sheath 32 without shortening it. This is also one of the present report purposes. Another acquired technical effect is a better control over the prosthesis radial expansion process procedure while coming out from inside sheath 32. The runners 42 involving the prosthesis 10 avoid the prosthesis sudden expansion.

The introducer and placer of repairs in tubulations, described in this report, consist in a tubular device, rigid or flexible, with external diameter smaller than the tubulation internal diameter; has in one of the ends, space to store the repair and has cables and stem in its interior with access through the other end, for handling and placement of repair in the tubulation.

The figures described below, show the main functional elements of the device. Besides not specifying the dimensions, they don't show the real proportionality of the elements that compose the device. The lack of proportionality in the drawings, is justified because the dimensions and proportion of the elements varies individually according to the application, size, and type of the repair and the tubulation's internal diameter.
The figure 1, shows the device with the ogive, the spacer tube, the scabbard, the maniple of the scabbard, the maniple of the spacer tube, the trigger and particular inside spot.
The figure 2 shows the particular inside spot, with the base of the ogive, the guide tube(3), the multilumem tube and the drag wires.
The figure 3 shows the base of the ogive with the chain holes of fitting and fixation of the wires
The figure 4 shows the guide tube, the multilumem tube and the drag wires
The figure 5 shows detail of the way of cramping the repair in the drag wires; the drag wires ends inside the chain holes in the base of the ogive; the repair involving the multilumem tube and stored inside the scabbard and aligned with the spacer tube. The repair has opening of arches for cramping and fixation in the wires.
The figure 6 shows the section, the scabbard, the maniple of the scabbard, the maniple of the spacer tube, and the trigger.
The figure 7 shows the section with the guide tube, the multilumem tube, the wires, and the spacer tube.
The figure 8 shows the section, the maniple of the spacer tube, the trigger with locking screw, and setting point of the wires in the trigger.
The figure 9 shows the detail with the multilumem tube, the wires, and the spacer tube.
The figure 10 shows constructive form where the multilumem tube is replaced by cylindrical tubing and connector; shows the base of the ogive with the chain holes, the guide tube, and the wires.
The figure 11 shows the base of the ogive with the connector set in the base. Shows the chaps of the connector.

The device is built according its employment, repair type and size , and internal diameter of the tubulation. Special attention is taken in the construction of the end that stores the repair 12. The repair 12 is built-in in the interior of the scabbard (7), involving the multilumem tube (4), occupying the empty of spacing tubing 6 and steed by the opening of eyelets 13 in the dragging wires 5. The device is introduced, in the tubulation being repaired, until the prosthesis 12 reaches the section of the tube where the defect is. The positioning can be previewed measuring the distance between the end and the defective section of the tube. The positioning of the repair of the repair can be monitored by X-ray, ultrasound and others. Once positioned the repair in the desired section, make the axial recoil of the sheath 7 sliding it axially in relation to the spacer tube (6), so the conjunct formed by the nose cone 1, core shaft 3, multilumem tube (4), the wires (5), spacer tube (6), and the prosthesis 12 remain still, liberating the prosthesis 12 from its niche. The prosthesis 12 goes through a self-expansion until it presses the internal wall of the tube; the prosthesis 12 is still set, through the opening of eyelets 13 in the wires (5). During this stage is possible to make an evaluation of the prosthesis 12 positioning related to the section of the tube; if it is necessary to make an adjustment, it is possible to displace axially the prosthesis 12 pushing the conjunct, and specially the nose cone 1, forward; the prosthesis 12 moves with the conjunct because of the drag of the wires (5) set in the opening of eyelets 13 of the prosthesis 12. After the adjustment, is bring into action the trigger (10) that gather the wires (5) inside the multilumem tube (4), disconnecting definitively the prosthesis 12 from the device.

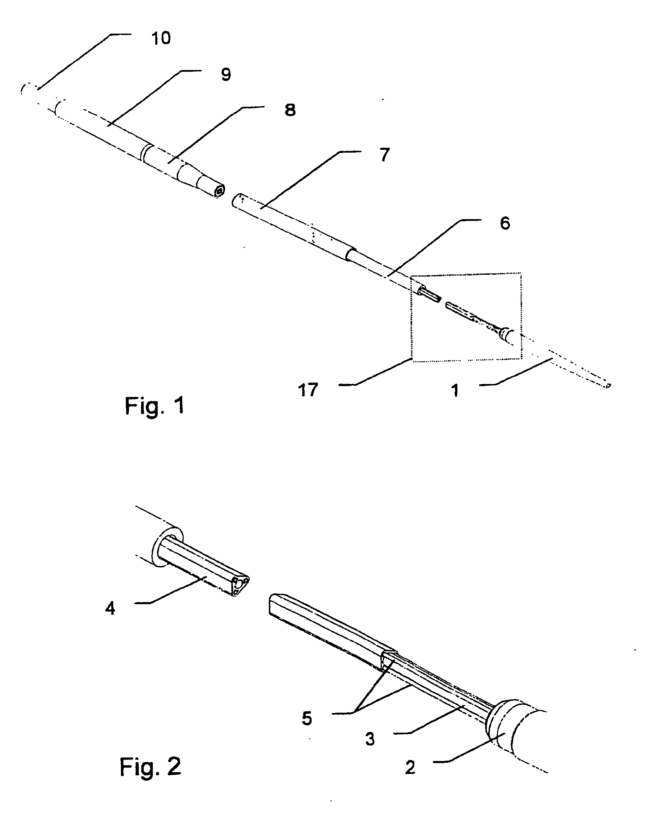

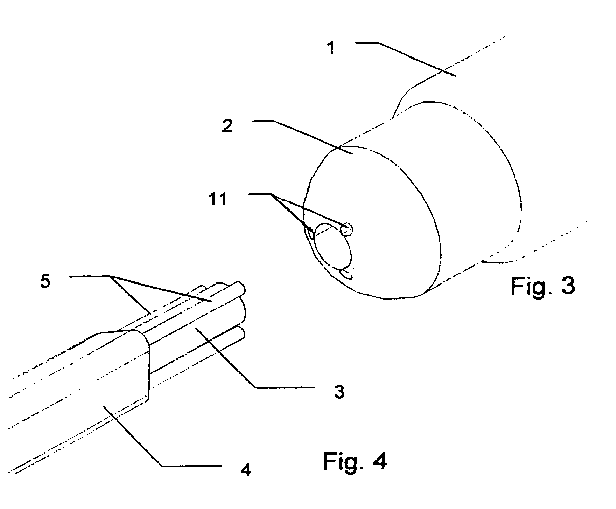

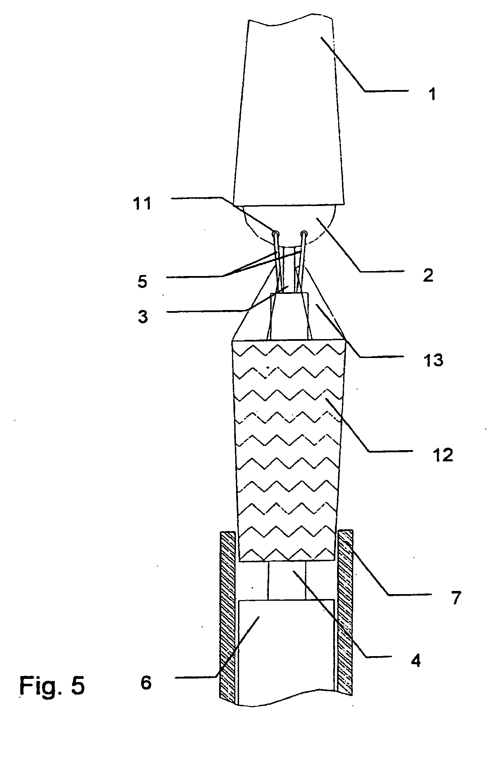

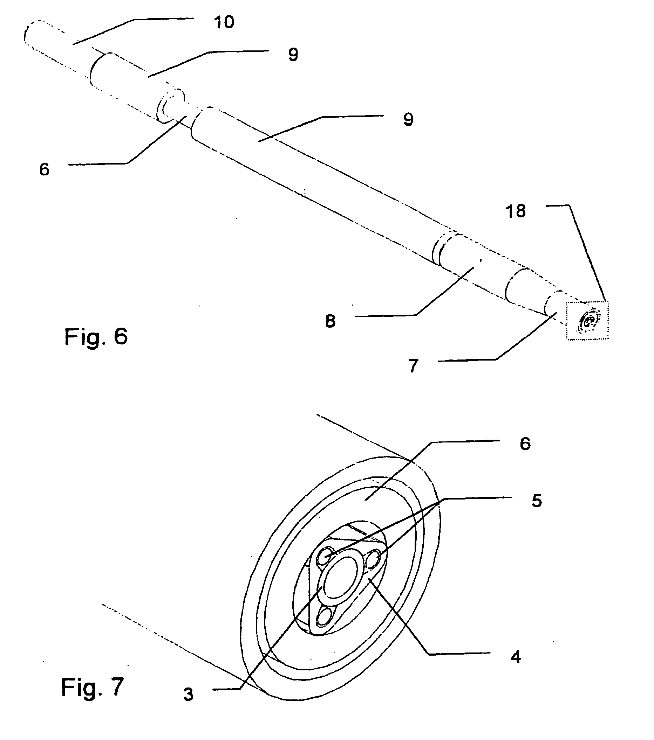

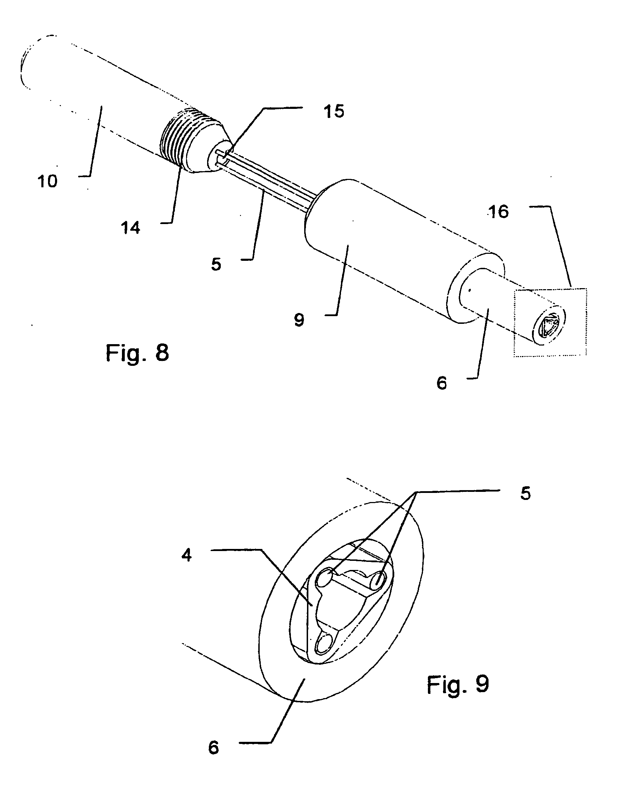

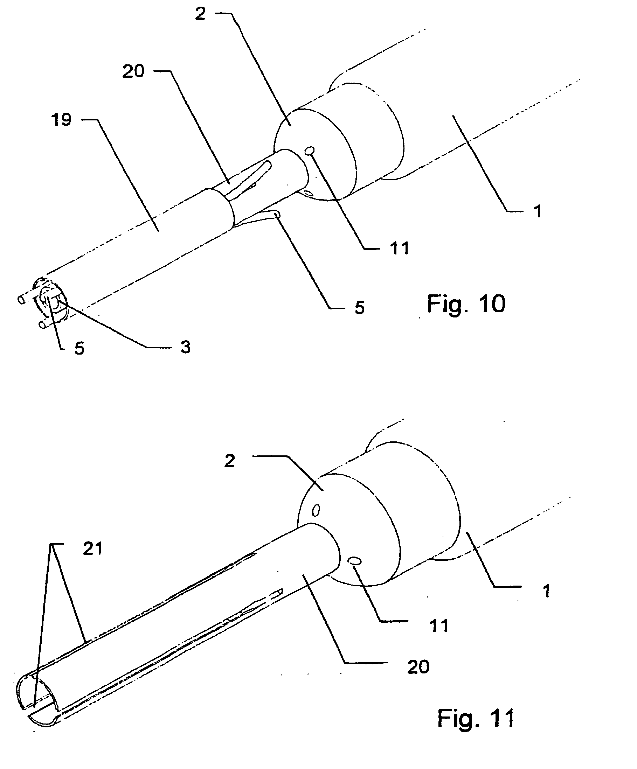

The multilumem tubing 4, showed in figures 2, 4, 5, 7, and 9 with straight section in triangular shape, may have the polygonal section with plurality of sides or rounded section. The shape of the section of the multilumem tube (4) depends on the constructive shape of the prosthesis 12, its bending and fittings. The figure 10 shows the tube (19) of rounded section, without longitudinal holes for the passage of the wires (5), replacing the multilumem tube (4). The dragging wires 5 run loose in the gap between the core shaft 3 and the flat tube (19); penetrate into the chaps (21) of the connector (20) and point to the scocket holes 11 of the base (2).

The axial recoil of the sheath 7 is obtained manually, keeping still the handle 9 of the spacer tube (6) and displacing axially the handle 8 of the sheath 7 in the direction of the handle 9 of the spacer tube (6).

The trigger (10) is a rigid stalk-cylindrical piece, placed in the end opposite to the nose cone 1; has a small cylindrical surface with external screw (14) that is screwed in the body of the handle 9 of the spacer tube (6).

The wires (5), that have one of the ends free to set in the opening of arch (13) of the prosthesis 12 and to penetrate in the scocket holes 11 of the nose cone 1, have the other extremity set in the base (15) of the trigger (10). Bring into action the trigger (10), consists in disconnecting (14) the trigger (10) from the maniple(9) of the spacer tube (6) and displace the trigger (10) axially away from the handle 9.

Although during the text and drawings, the scabbard is cited and represented as a cylindrical tube with uniform section, it may have geometrically the shape of a tube of scaled diameters. Part of the tube, next to the nose cone 1, may have the diameter larger than the sheath 7 body to store repairs that need big niches.

## Claims

1. A device suitable for placing a prosthesis into a tubing system comprising, from one of its ends,
- a nose cone (1) including a half spherical base (2),
- a central guide tube (3) extending from the half spherical base (2) of the nose cone (1),
- a plurality of dragging wires (5), extending from holes (11) in the half spherical base (2) of the nose cone (1),
- a multilumen tubing (4) being concentrically located around said central guide tube (3), the central guide tube (3) and the dragging wires being received and guided with independent axial translation in said multilumen tubing (4),
- a radially expandable prosthesis (12) being concentrically located around said central guide tube (3) and said multilumen tubing (4), provided with eyelets (13) connecting the prosthesis (12) to each dragging wire,
- a spacer tube (6) located concentrically around said multilumen tubing (4),
- a sheath (7) shaped as an external concentric tube concentrically covering said radially expandable prosthesis (12) and said spacer tube (6),
- a maniple (9) of the spacer tube (6) extending out of said sheath (7),
- a cylindrical trigger (10) provided with an external screw (14) which fits in a screw hole of said maniple (9) of spacer tube, the ends of the dragging wires (5) being fixed to said cylindrical trigger for pulling the prosthesis (12) from said sheath (7), which is axially movable with respect to the dragging wires (5).

2. The device according to claim 1; wherein holes (11) are radially allocated in the nose cone (1) and are equal to the number of dragging wires (5).

3. The device according to claim 2; wherein the cylindrical trigger (10) is detachable from the device for removing the dragging wires (5) from holes (11) of the nose cone (1) and the eyelets (13) of the prosthesis (12).

4. The device according to claim 3; further comprising a central guide tube (3) that extends through the prosthesis (12) and the sheath (7) and that comprises a distal end attached to the nose cone (1) and a proximal end that is attached to the maniple (9) of the spacer tube (6).

5. The device according to claim 4; wherein the trigger (10) that is removably attached to the handle of the central guide tube (3).

6. The device according to claim 5; wherein the multilumen tube (4) has a triangular flat section and a hole (11) in each apex of the triangular shape and other hole (11) at the centre.

## Patentansprüche

1. Zum Platzieren einer Prothese in ein Rohrsystem geeignete Vorrichtung, umfassend, von einem seiner Enden aus gesehen,
- eine Spitze (1) mit einer halbkugelförmigen Basis (2),
- ein zentrales Führungsrohr (3), das sich von der halbkugelförmigen Basis (2) der Spitze (1) erstreckt,
- eine Vielzahl von Zugdrähten (5), die sich aus Löchern (11) in der halbkugelförmigen Basis (2) der Spitze (1) erstrecken,
- ein Mehrfach-Lumen-Rohr (4), das konzentrisch um das zentrale Führungsrohr (3) platziert ist, wobei das zentrale Führungsrohr (3) und die Zugdrähte mit unabhängiger axialer Translation in dem Mehrfach-Lumen-Rohr (4) aufgenommen und geführt sind,
- eine radial ausdehnbare Prothese (12), die konzentrisch um das zentrale Führungsrohr (3) und das Mehrfach-Lumen-Rohr (4) platziert ist und mit Ösen (13) versehen ist, die die Prothese (12) mit jedem Zugdraht verbinden,
- ein Distanzrohr (6), das konzentrisch um das Mehrfach-Lumen-Rohr (4) herum platziert ist,
- eine Hülse (7), die als äußeres konzentrische Rohr geformt ist, das die radial ausdehnbare Prothese (12) und das Distanzrohr (6) konzentrisch abdeckt,
- einen Griff (9) des Distanzrohrs (6s), der sich aus der Hülse (7) heraus erstreckt,
- einen zylindrischen Auslöser (10), der mit einer äußeren Schraube (14) versehen ist, die in ein Schraubenloch des Griffs (9) des Distanzrohrs passt, wobei die Enden der Zugdrähte (5) an dem zylindrischen Auslöser befestigt sind, um die Prothese (12) aus der Hülse (7) zu ziehen, die axial in Bezug auf die Zugdrähte (5) beweglich ist.

2. Vorrichtung nach Anspruch 1, wobei Löcher (11) radial in der Spitze (1) angeordnet sind und ihre Anzahl gleich der Anzahl der Zugdrähte (5) ist.

3. Vorrichtung nach Anspruch 2, wobei der zylindrische Auslöser (10) von der Vorrichtung abnehmbar ist, um die Zugdrähte (5) aus den Löchern (11) der Spitze (1) und den Ösen (13) der Prothese (12) zu entfernen.

4. Vorrichtung nach Anspruch 3, weiter ein zentrales Führungsrohr (3) umfassend, das sich durch die Prothese (12) und die Hülse (7) erstreckt und das ein an der Spitze (1) angebrachtes distales Ende und ein am Griff (9) des Distanzrohrs (6) angebrachtes proximales Ende umfasst.

5. Vorrichtung nach Anspruch 4; wobei der Auslöser (10) entfernbar am Griff des zentralen Führungsrohrs (3) angebracht ist.

6. Vorrichtung nach Anspruch 5; wobei das Mehrfach-Lumen-Rohr (4) einen dreieckigen, flachen Querschnitt und ein Loch (11) in jeder Spitze der Dreiecksform und ein weiteres Loch (11) in der Mitte aufweist.

## Revendications

1. Dispositif approprié pour mettre en place une prothèse dans un système de tubes comprenant, à partir d'une de ses extrémités,
un cône (1) de nez incluant une base hémisphérique (2),
un tube central (3) de guidage s'étendant de la base hémisphérique (2) du cône (1) de nez,
une pluralité de fils (5) de traction, s'étendant à partir de trous (11) réalisés dans la base hémisphérique (2) du cône (1) de nez,
un tubage à lumières multiples (4) étant disposé concentriquement autour dudit tube central (3) de guidage, le tube central (3) de guidage et les fils de traction étant reçus et guidés selon une translation axiale indépendante dans ledit tubage à lumières multiples (4),
une prothèse pouvant se dilater radialement (12) étant disposée concentriquement autour dudit tube central (3) de guidage et dudit tubage à lumières multiples (4), pourvue d'oelllets (13) liant la prothèse (12) à chaque fil de traction,
un tube (6) d'écartement disposé concentriquement autour dudit tubage à lumières multiples (4),
un manchon protecteur (7) formé comme un tube extérieur concentrique couvrant concentriquement ladite prothèse pouvant se dilater radialement (12) et ledit tube (6) d'écartement,
une section (9) du tube (6) d'écartement s'étendant hors dudit manchon protecteur (7),
un dispositif à déclenchement cylindrique (10) doté d'une vis extérieure (14) qui s'ajuste dans un trou de vis de ladite section (9) du tube d'écartement, les extrémités des fils (5) de traction étant fixées audit dispositif à déclenchement cylindrique dans le but de tirer la prothèse (12) dudit manchon protecteur (7), qui est mobile axialement par rapport aux fils (5) de traction.

2. Dispositif selon la revendication 1, dans lequel des trous (11) réalisés dans le cône (1) de nez sont affectés radialement aux fils (5) de traction et leur sont égaux en nombre;

3. Dispositif selon la revendication 2, dans lequel le dispositif à déclenchement cylindrique (10) peut être désolidarisé du: dispositif pour retirer les fils (5) de traction des trous (11) du cône (1) de nez et des oeillets (13) de la prothèse (12).

4. Dispositif selon la revendication 3, comprenant en outre un tube central (3) de guidage qui s'étend à travers la prothèse (12) et le manchon protecteur (7) et dont une extrémité distale est assujettie au cône (1) de nez et dont une extrémité proximale est assujettie à la section (9) du tube (6) d'écartement.

5. Dispositif selon la revendication 4, dans lequel le dispositif à déclenchement (10) est assujetti de façon amovible à la poignée du tube central (3) de guidage.

6. Dispositif selon la revendication 5, dans lequel le tube à lumières multiples (4) comporte une section triangulaire plate et un trou (11) réalisé dans chaque sommet de la forme triangulaire et un autre trou (11) réalisé au niveau du centre.
